# EUROPÄISCHE PATENTSCHRIFT

(11) **EP 0 314 027 B1**
(45) Veröffentlichungstag und Bekanntmachung des Hinweises auf die Patenterteilung: **19.05.1993**
(21) Anmeldenummer: 88117637.4
(22) Anmeldetag: 22.10.1988
(51) Int. Cl.: A61B 5/00

(54) **Verfahren und Vorrichtung zum Betreiben und Kalibrieren mehrerer Fühler für biologische und physiologische Messwerte**
Method and device for activating and calibrating several sensors for biological and physiological parameters
Méthode et dispositif d'actionnement et d'étalonnage de plusieurs capteurs de grandeurs biologiques et physiologiques

(30) Priorität: 29.10.1987 DE 3736678
(43) Veröffentlichungstag der Anmeldung: 03.05.1989
(73) Patentinhaber: Drägerwerk Aktiengesellschaft, 23542 Lübeck (DE)
(72) Erfinder: Hölscher, Uvo, Dr., D-2406 Stockelsdorf (DE)

(56) Entgegenhaltungen:
- EP-A- 0 074 498
- DE-A- 2 255 382
- DE-A- 2 906 201
- DE-A- 2 929 387

## Beschreibung

Die Erfindung betrifft ein Verfahren nach dem Oberbegriff des Anspruchs 1 und eine Vorrichtung zur Durchführung des Verfahrens.

Biologische oder physiologische Meßwerte können über einen oder mehrere Meßfühler aufgenommen werden, welche zum Zwecke der Meßwerterfassung beispielsweise elektrische oder elektromagnetische Energie oder Wärmeenergie in die Oberfläche des zu untersuchenden Meßobjektes einspeisen müssen. Hierzu gehören beispielsweise die Laser-Doppler-Geschwindigkeitsmessung des Blutes oder die transcutane Bestimmung von Blutgaswerten, bei welchen die menschliche Haut mit Wärmeenergie versorgt werden muß.

Für die transkutane Bestimmung des Partialdruckes von Sauerstoff im Blut wird dazu eine Anordnung von mehreren beheizbaren Meßfühlern in einem gemeinsamen Meßkopf eingesetzt, welche nach einer vorbestimmten Zykluszeit zur Messung herangezogen werden, wobei einer der Fühler beheizt wird und die Meßwerte aufnimmt und die übrigen Fühler während dieser Zeit ausgeschaltet bleiben. Nach Ablauf des vorgegebenen Meßzyklus wird der aktuelle Meßfühler abgeschaltet und einer der übrigen vorher ausgeschalteten Fühler eingeschaltet.

Während des Betriebes des Meßfühlers wird der aufgenommene Meßwert über eine Auswerte- und Anzeigeeinheit dargestellt. Die für die Messung notwendige Wärmeenergie wird ebenfalls über die Auswerte- und Anzeigeeinheit an den jeweilig messenden Meßfühler abgegeben, der sie dann während der Meßzeit auf die Hautoberfläche überträgt.

Ein solches Verfahren und eine dazugehörige Meßeinrichtung wird in der DE-OS 29 06 201 beschrieben.

Bei dem bekannten Verfahren sind mindestens drei Meßfühler in einem gemeinsamen Meßkopf angeordnet, wobei jeder einzelne Meßfühler nach einem vorgegebenen Zeittakt zyklisch ein- und ausgeschaltet wird. Während der Einschaltdauer eines jeden Meßfühlers wird der von ihm gemessene Partialdruck von Sauerstoff im Blut transcutan erfaßt und angezeigt.

Nachteilig bei dem bekannten Verfahren ist es, daß während der Meßzeit des einen Meßfühlers die übrigen ausgeschaltet sind, und daß zwischen dem Ausschalten des ersten Meßfühlers und dem Einschalten des nächsten Meßfühlers ein Zeitraum vergeht, so daß die aufeinanderfolgenden Meßwerte nicht kontinuierlich angezeigt werden, sondern ein ständiges Auf und Ab der Meßwerte zu beobachten ist.

Während eines länger andauernden Meßzyklus ist es notwendig, die einzelnen Meßfühler zu kalibrieren oder die einzelnen Meßfühler untereinander in ihrer Signalempfindlchkeit aufeinander abzustimmen (Normierung). Jeder einzelne Meßfühler besitzt nämlich eine ihm eigene Charakteristik, die sich im Driftverhalten äußern, oder die durch sich verändernde Meßflächen oder auch Elektrolyt-Zusammensetzungen den Meßwert beeinflussen. Darüberhinaus beeinflussen Hautveränderungen unter jedem Sensor in unterschiedlicher Weise das Meßverhalten. Zu diesem Zweck müssen die bekannten Meßfühler von dem Meßobjekt entfernt und einer getrennten Kalibration bzw. Normierung zugeführt werden. Während dieses Zeitabschnittes ist eine Messung nicht möglich, so daß die erforderliche kontinuierliche Überwachung nicht sichergestellt ist. Beim Einsatz mehrerer Meßfühler ist der Zeitaufwand für die Kalibration bzw. Normierung erheblich, und die nachfolgende Einlaufzeit vor Beginn einer erneuten Messung kann nicht vernachlässigt werden.

Der vorliegenden Erfindung liegt somit die Aufgabe zugrunde, ein Verfahren zum gleichzeitigen oder abwechselnden Betreiben mehrerer Fühler der genannten Art so zu verbessern, daß eine stetige Meßwertanzeige auch während einer Kalibration oder Normierung der Meßfühler möglich ist.

Zur Lösung der Aufgabe ist vorgesehen, daß, zum Zweck der stetigen Fortsetzung der Meßwertanzeige des Meßfühlers durch den Bereitschaftsfühler, nach dem Überführen des Bereitschaftsfühlers in seine Meßphase das aktuelle Meßsignal des immer noch in der Meßphase arbeitenden Meßfühlers zur Normierung des aktuellen Meßsignales des Bereitschaftsfühlers herangezogen wird, wonach der Meßfühler in die Bereitschaftsphase gebracht und als neuer Bereitschaftsfühler betrieben wird.

Der Vorteil der Erfindung liegt im wesentlichen darin, daß der Meßfühler zumindest so lange noch seine Meßwerte weiterhin aufnimmt, bis der Bereitschaftsfühler in die Meßphase gebracht worden ist, und daß die beiden Meßsignale des immer noch messenden Meßfühlers und des schon in die Meßphase gebrachten Bereitschaftsfühlers von der Auswerteeinheit zur Normierung des Meßsignals des Bereitschaftsfühlers verarbeitet wird. Danach ist das Meßsignal des Bereitschaftsfühlers auf das vorangegangene Meßsignal des Meßfühlers normiert, und die Anzeige des Meßwertes kann von dem jetzt als Meßfühler arbeitenden Bereitschaftsfühler kontinuierlich übernommen werden. Erst jetzt wird der Meßfühler in seine Bereitschaftsphase umgeschaltet, so daß eine kontinuierliche und stetige Fortsetzung der Meßwertanzeige ermöglicht ist.

Als Meßphase wird in dem Falle der transcutanen Bestimmung von Sauerstoff-Partialdruck im Blut die Zeit angesehen, während welcher der Fühler seine Betriebstemperatur erreicht hat und eine mehr oder weniger konstante Meßwertanzeige ersichtlich ist. Als Bereitschaftsphase wird derjenige Zeitraum angesehen, während der der entsprechende Fühler auf einer niedrigeren Bereitschaftstemperatur gehalten wird.

Die während einer kontinuierlichen Messung durch verschiedene Meßfühler notwendige Normierung kann daher vorgenommen werden, ohne daß die Meßwertanzeige sprunghaft geändert wird.

In gewissen Zeitabständen ist eine Kalibrierung der Meßfühler notwendig, bei denen die Meßfühler einem Kalibriergas bekannter Zusammensetzung ausgesetzt werden. Ohne die Messung dabei zu unterbrechen, ist die Kalibrierung dadurch durchführbar, daß nach erfolgter Normierung zunächst einer der in der Meßphase arbeitenden Fühler zur Übernahme von neuen Kalibrierwerten an eine Kalibriereinheit angeschlossen wird, und diese Kalibrierwerte von dem anderen, nicht an die Kalibriereinheit angeschlossenen Meßfühler zur Korrektur seiner eigenen, schon vorhandenen Kalibrierwerte übernommen werden. Erst danach wird einer der Meßfühler in seine Bereitschaftsphase gebracht und der kalibrierte Meßfühler übernimmt die Messung. Ein vollständiger Kalibriervorgang läuft dann in folgenden Schritten ab: Der Bereitschaftsfühler wird in die Meßphase gebracht, seine Meßsignale anhand des immer noch in der Meßphase arbeitenden Meßfühlers normiert. Anschließend wird ein Meßfühler kalibriert und die Meßsignale an den zweiten Meßfühler übertragen, so daß dessen normierte Meßwerte durch Übernahme der Kalibrierwerte des ersten Meßfühlers kalibriert werden. Danach befindet sich der ehemalige Bereitschaftsfühler endgültig in der Meßphase, und der erste Meßfühler wird in die Bereitschaftsphase umgeschaltet. Somit erhält man für die nachfolgende Messung einsetzbare Meß- und Bereitschaftsfühler.

Die Kalibration kann nicht nur dadurch ausgeführt werden, daß einer der in der Meßphase betriebenen Fühler einem Kalibriergas bekannter Zusammensetzung ausgesetzt wird, sondern die Kalibrierwerte können über eine Eingabeeinheit an die Versorgungs- und Auswerteeinheit beispielsweise anhand einer separat durchgeführten Blutgasanalyse eingegeben werden. Diese eingegebenen Kalibrierwerte werden von dem zu kalibrierenden Fühler übernommen sowie an den in der Meßphase arbeitenden Bereitschaftsfühler übertragen.

Zweckmäßigerweise erfolgt die Normierung durch Bildung des Quotienten oder der Differenz aus den absoluten, durch Exponentbildung verrechneten oder delogarithmierten Meßsignalen des Meßfühlers und des Bereitschaftsfühlers. Ein derartiges Normierungsverfahren ist besonders dann günstig, wenn die Fühler zur transcutanen Bestimmung des Sauerstoff-oder CO₂-Partialdruckes des Blutes eingesetzt werden.

Vorteilhafterweise werden die verarbeiteten Meßsignale der Fühler an einer gemeinsamen Sichtanzeige als Meßwerte dargestellt. Die stetige Erfassung der Meßsignale wird dadurch auch an einer gemeinsamen Anzeigeeinheit als Meßwert angezeigt, wodurch dem Anwender ersichtlich wird, daß trotz mehrerer Fühler eine für alle gültige Meßwertanzeige möglich ist.

Eine Vorrichtung zum gleichzeitigen oder abwechselnden Betreiben mehrerer Fühler für biologische und physiologische Meßwerte besteht aus einer Auswerte-und Anzeigeeinheit, welche eine Übernahmeeinheit zur Übernahme der Meßsignale des Meßfühlers und des Bereitschaftsfühlers, eine Verarbeitungseinheit zur Normierung der Meßsignale des Bereitschaftsfühlers sowie eine Übergabeeinheit zur Übergabe des Normierungswertes an die Auswerteeinheit des Bereitschaftsfühlers aufweist. Für die Normierung der sich abwechselnden Fühler werden ihre Meßsignale aufgenommen, zur Normierung verarbeitet und das normierte Meßsignal des Meßfühlers an den in der Meßphase befindlichen Bereitschaftsfühler abgegeben. Danach erfolgt die Messung über den normierten Bereitschaftsfühler, der als neuer Meßfühler eingesetzt wird.

Zum Zwecke der Kalibrierung ist vorteilhafterweise an die Auswerteeinheit eine Kalibriereinheit angeschlossen. Die Kalibriereinheit setzt den zu kalibrierenden Fühler einem Kalibriergas bekannter Zusammensetzung aus, wodurch die kalibrierten Meßsignale dem in der Meßphase befindlichen Bereitschaftsfühler übertragen werden.

Der Auswerte- oder Kalibriereinheit kann eine numerische Eingabeeinheit zugeordnet sein, über welche beispielsweise durch ein numerisches Tastenfeld Kalibrierwerte eingegeben werden können, die beispielsweise durch eine Blutgasanalyse gewonnen wurden. Dadurch brauchen die zu kalibrierenden Fühler noch nicht einmal von dem Meßobjekt entfernt zu werden.

Zweckmäßigerweise besitzt die Auswerteeinheit eine gemeinsame Sichtanzeige für die Meßwerte sowohl des Meßfühlers als auch des Bereitschaftsfühlers.

Zur Durchführung der Normierung ist eine Normierungseinheit vorgesehen, in welcher über ein Rechenwerk die absoluten, durch Exponentialfunktion verrechneten oder delogarithmierten Meßsignale der Fühler durch Quotienten- oder Differenzbildung zu Meßwerten verarbeitbar sind. Bei einem linearen Zusammenhang zwischen Meßsignal und Meßgröße können die absoluten Meßsignale herangezogen werden, bei einem logarithmischen Verlauf von Meßsignal und Meßgröße sind die Meßsignale vorher zu delogarithmieren, bevor sie durch Quotienten- oder Differenzbildung zu Meßwerten weiterverarbeitet werden.

Das Verfahren wird anhand eines schematischen Ablaufdiagramms und die Vorrichtung zur Durchführung des Verfahrens wird beispielhaft in der Zeichnung dargestellt und im folgenden näher erläutert.

Es zeigen
- Figur 1: eine schematische Darstellung des Meßsignalverlaufes und der Anzeige der Meßwerte mit Normierung
- Figur 2: eine Darstellung nach Fig. 1 mit zusätzlicher Kalibrierung
- Figur 3: ein Blockdiagramm einer Auswerte- und Anzeigeeinheit.

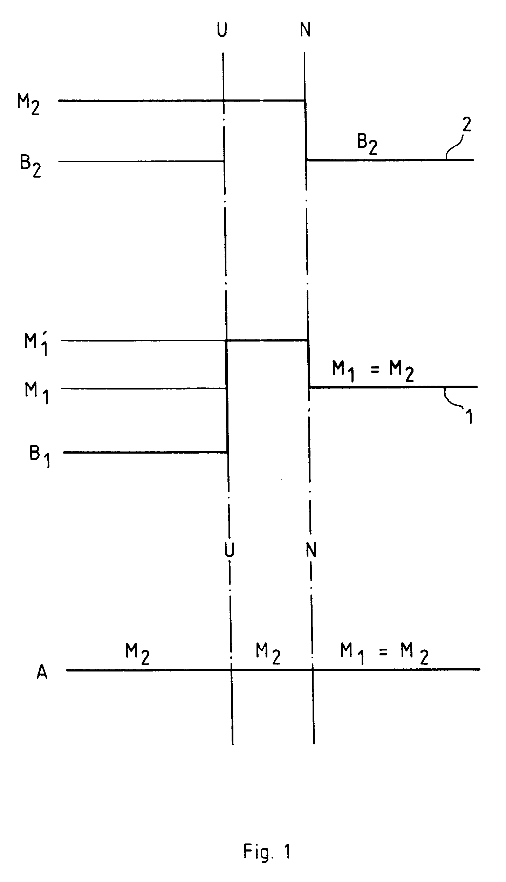

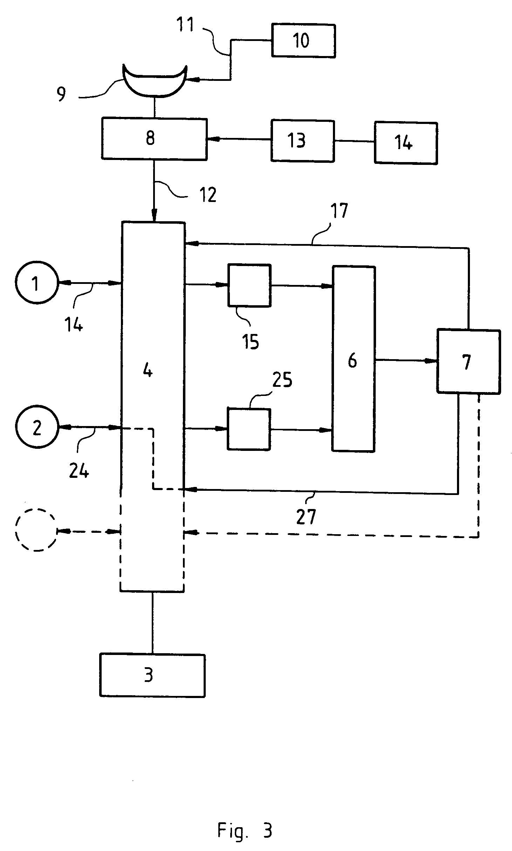

In Figur 1 ist der relative Verlauf der Meßsignale während der Durchführung des Verfahrens beispielhaft an zwei Fühlern dargestellt, von denen einer als Bereitschaftsfühler (1) und ein anderer als Meßfühler (2) eingesetzt sind. In der Bereitschaftsphase B₁ des Bereitschaftsfühlers (1) befindet sich der Meßfühler (2) in der Meßphase M₂, wobei die Anzeige A durch eine Sichtanzeige (3) (vergleiche Figur 3) dargestellt wird. Nach einem vorgebbaren Umschaltpunkt U wird der Bereitschaftsfühler (1) aus seiner Bereitschaftsphase B₁ in seine Meßphase M₁′ überführt und so zur Messung vorbereitet. Sein Meßsignal M₁′ stimmt in der Regel nicht mit dem Meßsignal M₂ des Meßfühlers (2) überein. Eventuelle Abweichungen der Meßsignale M₁′ und M₂ werden durch eine Normierung N ausgeglichen, so daß das Meßsignal M₁′ des Bereitschaftsfühlers (1) auf das Meßsignal M₁ absinkt, welches nunmehr mit dem Meßsignal M₂ des Meßfühler (2) übereinstimmt. Nach durchgeführter Normierung N wird der Meßfühler (2) in seiner Bereitschaftsphase B₂ betrieben. Die Anzeige A an der Sichtanzeige (3) stellt nach der Umschaltung U immer noch den Meßwert M₂ des Meßfühlers (2) dar, und nach durchgeführter Normierung erfolgt die Anzeige des Meßwertes M₂ des in der Meßphase befindlichen ehemaligen Bereitschaftsfühlers (1), welcher mit dem Meßwert M₂ des ehemaligen Meßfühlers (2) übereinstimmt. Somit erhält man am Umschaltpunkt N eine stetige Anzeige A der Meßwerte M₁, M₂, deren Schwankungen nur noch durch unterschiedlich aufgenommene Meßgrößen, nicht mehr jedoch durch nichtphysiologische Meßwertverschiebungen der einzelnen Fühler (1, 2) bedingt sind.

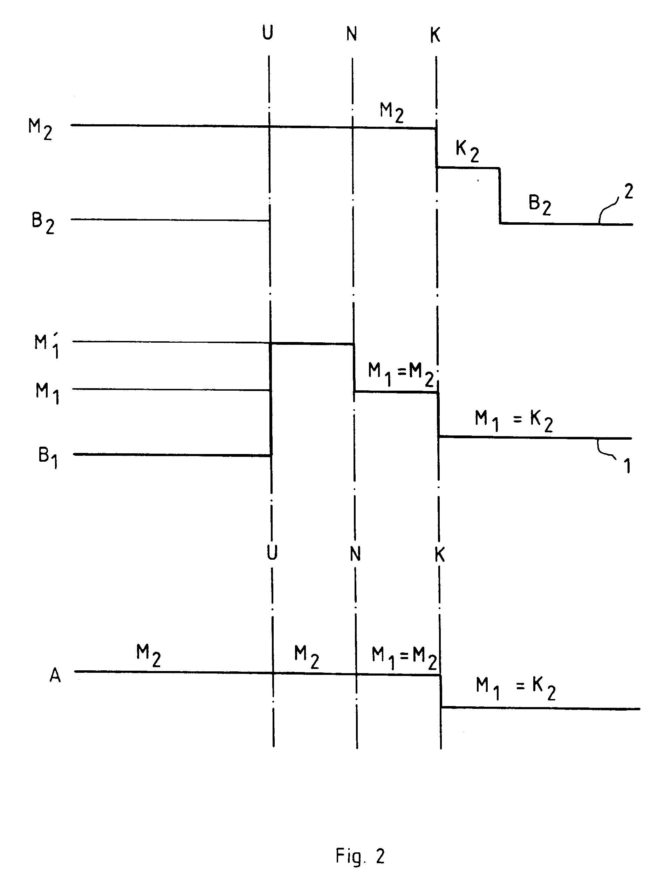

In Figur 2 ist der Verlauf der Meßsignale und der Meßwerte ähnlich wie in Figur 1 dargestellt, wobei jedoch nach erfolgter Normierung N eine Kalibrierung K des Meßfühlers (2) durchgeführt wird. Gleiche Signalverläufe sind durch gleiche Buchstaben- und Ziffernkennzeichnung dargestellt. Nach erfolgter Normierung N wird das Meßsignal M₂ des Meßfühlers (2) kalibriert (K), so daß ein kalibriertes Meßsignal K₂ entsteht, welches vom Bereitschaftsfühler (1) übernommen wird. Dessen Meßsignal M₁ stimmt danach mit dem kalibrierten Meßsignal K₂ des Meßfühlers (2) überein. Der Meßfühler (2) wird nunmehr in seine Bereitschaftsphase B₂ überführt, und die Anzeige A des Meßwertes M₁ des Bereitschaftsfühlers (1) entspricht dem kalibrierten Meßsignal K₂ des ehemaligen Meßfühlers (2). Auch in diesem Falle wird eine stetige Anzeige A der Meßwerte ermöglicht, wobei eine Änderung des Meßwertes infolge der Kalibrierung K lückenlos übernommen wird.

In Figur 3 sind der Bereitschaftsfühler (1) und der Meßfühler (2) an eine Versorgungs- und Auswerteeinheit (4) angeschlossen, welche über Versorgungsleitungen (14, 24) mit den Fühlern (1, 2) verbunden ist. In den Versorgungsleitungen (14, 24) findet sowohl die Versorgung der Fühler (1,2) mit zu ihrem Betreiben notwendiger Energie, als auch die Übertragung der Meßsignale zur Weiterverarbeitung statt. Die Weiterverarbeitung der Meßsignale erfolgt über den jeweiligen Fühlern (1, 2) zugeordnete bernahmeeinheiten (15, 25). Die übernommenen Meßsignale werden in einer Verarbeitungseinheit (6) zum Zwecke der Normierung verarbeitet und die normierten Meßsignale an den jeweiligen zu normierenden Fühler (1, 2) über eine Übergabeeinheit (7) weitergeleitet. Die Übergabeeinheit (7) ist mit entsprechenden Übergabeleitungen (17, 27) an die Versorgungseinheit (4) angeschlossen, über welche die normierten Meßsignale als Grundlage für die nachfolgende Messung eingesetzt werden. An die Versorgungseinheit (4) ist sowohl eine für die Fühler (1, 2) gemeinsame Sichtanzeige (3) und eine Kalibriereinheit (8) angeschlossen. Zum Zwecke der Kalibrierung kann einer der zu kalibrierenden Fühler (1, 2) an eine Kalibrieraufnahme (9) angeschlossen werden, die mit einer Prüfgasquelle (10) über eine Prüfgasleitung (11) verbunden ist. Während der Kalibrierung wird der zu kalibrierende Fühler (1, 2) mit dem Prüfgas aus der Prüfgasquelle (10) beaufschlagt und die Kalibrierwerte über eine Kalibrierleitung (12) der Versorgungseinheit (4) zugeleitet. Zur Kalibrierung können die erforderlichen Kalibrierwerte auch über eine separate Eingabeeinheit (13) an die Kalibriereinheit (8) und Versorgungseinheit (4) weitergegeben werden, wozu ein numerisches Dateneingabefeld (14) vorgesehen ist.

In der Figur 3 sind beispielhaft zwei Fühler (1, 2) an die Versorgungseinheit (4) angeschlossen. Es ist jedoch auch möglich, ohne an dem Verfahren oder der zur Durchführung des Verfahrens notwendigen Vorrichtung etwas zu verändern, weitere Sensoren an die Versorgungseinheit (4) anzuschließen.

## Patentansprüche

1. Verfahren zum gleichzeitigen oder abwechselnden Betreiben mehrerer Fühler für biologische und physiologische Meßwerte, insbesondere für die transcutane Bestimmung des Druckes von Gasen im Blut, wobei mehrere Fühler (1,2) an eine gemeinsame Auswerte- und Anzeigeeinheit (3,4) angeschlossen sind, von denen zumindest zeitweise einer der Fühler als Meßfühler (2) in einer Meßphase und ein anderer Fühler als Bereitschaftsfühler (1) in einer Bereitschaftsphase betrieben werden, dadurch gekennzeichnet, daß, zum Zweck der stetigen Fortsetzung der Meßwertanzeige des Meßfühlers (2) durch den Bereitschaftsfühler (1), nach dem Überführen des Bereitschaftsfühlers (1) in seine Meßphase das aktuelle Meßsignal des immer noch in der Meßphase arbeitenden Meßfühlers (2) zur Normierung des aktuellen Meßsignales des Bereitschaftsfühlers (1) herangezogen wird, wonach der Meßfühler (2) in die Bereitschaftsphase gebracht und als neuer Bereitschaftsfühler (1) betrieben wird.

2. Verfahren nach Anspruch 1, dadurch gekennzeichnet, daß nach erfolgter Normierung zunächst einer der in der Meßphase arbeitenden Fühler (1, 2) zur Übernahme von neuen Kalibrierwerten an eine Kalibriereinheit (8) angeschlossen wird und diese neuen Kalibrierwerte von dem anderen, nicht an die Kalibriereinheit angeschlossenen Meßfühler (2) zur Korrektur seiner eigenen schon vorhandenen Kalibrierwerte übernommen werden.

3. Verfahren nach Anspruch 1, dadurch gekennzeichnet, daß zur Kalibration eines der in der Meßphase arbeitenden Fühler (1, 2) die Kalibrierwerte über eine Eingabeeinheit (13) an die Versorgungs- und Auswerteeinheit (4) übertragen werden.

4. Verfahren nach einem der Ansprüche 1 bis 3, dadurch gekennzeichnet, daß die Normierung unter Bildung des Quotienten oder der Differenz aus den absoluten, durch Exponentialfunktion verrechneten oder delogarithmierten Meßsignalen des Meßfühlers (2) und des Bereitschaftsfühlers (1) erfolgt.

5. Verfahren nach einem der Ansprüche 1 bis 4, dadurch gekennzeichnet, daß die verarbeiteten Meßsignale der Fühler (1, 2) an einer gemeinsamen Sichtanzeige (3) als Meßwerte dargestellt werden.

6. Vorrichtung zum gleichzeitigen oder abwechselnden Betreiben mehrerer Fühler für biologische und physiologische Meßwerte nach dem Verfahren nach Anspruch 1, dadurch gekennzechnet, daß die Auswerte- und Anzeigeeinheit eine Übernahmeeinheit (15, 25) zur Übernahme der Meßsignale des Meßfühlers (1) und des Bereitschaftsfühlers (2), eine Verarbeitungseinheit (6) zur Normierung der Meßsignale des Bereitschaftsfühlers (1) sowie eine Übergabeeinheit (7) zur Übergabe des Normierungswertes an die Versorgungs- und Auswerteeinheit (4) des Bereitschaftsfühlers (1) aufweist.

7. Vorrichtung nach Anspruch 6, dadurch gekennzeichnet, daß eine Kalibriereinheit (8) zur Übertragung der Kalibrierwerte an die Versorgungs- und Auswerteeinheit (4) vorgesehen ist.

8. Vorrichtung nach Anspruch 7, dadurch gekennzeichnet, daß die Versorgungs- und Auswerteeinheit (4) oder Kalibriereinheit (8) eine numerische Eingabeeinheit (13) zur Eingabe der zu übertragenden Kalibrierwerte besitzt.

9. Vorrichtung nach einem der Ansprüche 6 bis 8, dadurch gekennzeichnet, daß für die Darstellung der Meßwerte sowohl des Meßfühlers (2) als auch des Bereitschaftsfühlers (1) eine gemeinsame Sichtanzeige (3) vorgesehen ist.

10. Vorrichtung nach einem der Ansprüche 6 bis 8, dadurch gekennzeichnet, daß zur Durchführung der Normierung in der Verarbeitungseinheit (6) die absoluten, durch Exponentialfunktion verrechneten oder delogarithmierten Meßsignale der Fühler (1, 2) durch Quotienten- oder Differenzbildung zu Meßwerten verarbeitbar sind.

## Claims

1. Method for the simultaneous or alternate operation of several sensors for biological and physiological measured values, in particular for the transcutaneous determination of the pressure of gases in blood, whereby several sensors (1, 2) are connected to a common evaluation and display unit (3, 4), of which at least for a time one of the sensors is operated as measuring sensor (2) in a measuring phase and another sensor as stand-by sensor (1) in a stand-by phase, characterized in that for the purpose of steady continuation of the measured-value display of the measuring sensor (2) by means of the stand-by sensor (1), after the transfer of the stand-by sensor (1) into its measuring phase, the actual measured signal of the measuring sensor (2) still working in the measuring phase is used for standardizing the actual measured signal of the stand-by sensor (1), after which the measuring sensor (2) is brought into the stand-by phase and is operated as new stand-by sensor (1).

2. Method according to claim 1, characterized in that after the standardization has taken place, first of all one of the sensors (1, 2) working in the measuring phase is connected to a calibration unit (8) for the acceptance of new calibration values and these new calibration values are accepted by the other measuring sensor (2), not connected to the calibration unit, for the correction of its own calibration values already existing.

3. Method according to claim 1, characterized in that for the calibration of one of the sensors (1, 2) working in the measuring phase the calibration values are transferred by way of an input unit (13) to the supply and evaluation unit (4).

4. Method according to one of claims 1 to 3, characterized in that the standardization takes place with the formation of the quotient or the difference of the measured signals of the measuring sensor (2) and of the stand-by sensor (1), the measured signals being absolute, compensated through exponential function or delogarithmized.

5. Method according to one of claims 1 to 4, characterized in that the processed measured signals of the sensors (1, 2) are represented as measured values on a common visual display (3).

6. Device for the simultaneous or alternate operation of several sensors for biological and physiological measured values according to the method according to claim 1, characterized in that the evaluation and display unit has an acceptance unit (15, 25) for accepting the measured signals of the measuring sensor (1) and of the stand-by sensor (2), a processing unit (6) for standardizing the measured signals of the stand-by sensor (1) and a delivery unit (7) for delivering the standardization value to the supply and evaluation unit (4) of the stand-by sensor (1).

7. Device according to claim 6, characterized in that a calibration unit (8) is provided for transferring the calibration values to the supply and evaluation unit (4).

8. Device according to claim 7, characterized in that the supply and evaluation unit (4) or calibration unit (8) has a numerical input unit (13) for the input of the calibration values to be transferred.

9. Device according to one of claims 6 to 8, characterized in that a common visual display (3) is provided for the representation of the measured values both of the measuring sensor (2) and of the stand-by sensor (1).

10. Device according to one of claims 6 to 8, characterized in that to carry out the standardization in the processing unit (6) the measured signals of the sensors (1, 2), which measured signals are absolute, compensated through exponential function or delogarithmized, can be processed into measured values through quotient or differential formation.

## Revendications

1. Procédé pour la mise en service simultanée ou alternée de plusieurs capteurs de valeurs de mesures biologiques et physiologiques, en particulier pour la détermination transcutanée de la pression de gaz dans le sang, plusieurs capteurs (1, 2) étant raccordés à une unité d'analyse et d'affichage (3, 4) commune, dont, au moins temporairement, l'un des capteurs fonctionne en tant que capteur de mesure (2) dans une phase de mesure et un autre capteur comme capteur d'attente (1), dans une phase d'attente, caractérisé en ce qu'en vue de la poursuite permanente de l'affichage des valeurs de mesure du capteur de mesure (2) par le capteur d'attente (1), après passage du capteur d'attente (1) dans sa phase de mesure, le signal de mesure actuel du capteur de mesure (2), fonctionnant toujours dans la phase de mesure, est utilisé pour normaliser le signal de mesure actuel du capteur d'attente (1), après quoi le capteur de mesure (2) est amené dans la phase d'attente et fonctionne comme un nouveau capteur d'attente (1).

2. Procédé selon la revendication 1, caractérisé en ce qu'une fois la normalisation effectuée, l'un des capteurs (1, 2) fonctionnant en phase de mesure est d'abord raccordé à une unité de calibrage (8), pour la prise de nouvelles valeurs de calibrage, et ces nouvelles valeurs de calibrage sont reprises par l'autre capteur de mesure (2), non raccordé à l'unité de calibrage, en vue de corriger ses propres valeurs de calibrage existantes.

3. Procédé selon la revendication 1, caractérisé en ce que pour le calibrage de l'un des capteurs (1, 2), fonctionnant en phase de mesure, les valeurs de calibrage sont transmises à l'unité d'alimentation et d'analyse (4), par une unité de saisie (13).

4. Procédé selon l'une des revendications 1 à 3, caractérisé en ce que la normalisation s'effectue par formation du quotient ou de la différence des signaux de mesure absolus, calculés par fonction exponentielle ou délogarithmés du capteur de mesure (2) et du capteur d'attente (1).

5. Procédé selon l'une des revendications 1 à 4, caractérisé en ce que les signaux de mesure traités, des capteurs (1, 2), sont représentés sur une unité d'affichage (3) commune en tant que valeurs de mesure.

6. Procédé pour la mise en service simultanée ou alternée de plusieurs capteurs de valeurs de mesures biologiques et physiologiques selon le procédé de la revendication 1, caractérisé en ce que l'unité d'analyse et d'affichage comporte une unité de reprise (15, 25) pour la reprise des signaux de mesure du capteur de mesure (1) et du capteur d'attente (2), une unité de traitement (6) pour normaliser les signaux de mesure du capteur d'attente (1) ainsi qu'une unité de transfert (7) pour transférer la valeur de normalisation à l'unité d'alimentation et d'analyse (4) du capteur d'attente (1).

7. Dispositif selon la revendication 6, caractérisé en ce qu'il est prévu une unité de calibrage (8) pour transmettre les valeurs de calibrage à l'unité d'alimentation et d'analyse (4).

8. Dispositif selon la revendication 7, caractérisé en ce que l'unité d'alimentation et d'analyse (4) ou l'unité de calibrage (8) possède une unité de saisie (13) numérique pour introduire les valeurs de calibrage à transmettre.

9. Dispositif selon l'une des revendications 6 à 8, caractérisé en ce qu'il est prévu une unité d'affichage (3) commune pour représenter les valeurs de mesure du capteur de mesure (2) ainsi que du capteur d'attente (1).

10. Dispositif selon l'une des revendications 6 à 8, caractérisé en ce que pour procéder à la normalisation dans l'unité de traitement (6), les signaux de mesure absolus, calculés par fonction exponentielle ou délogarithmés des capteurs (1, 2), sont transformés en valeurs de mesure par formation d'un quotient ou d'une différence.
